# EUROPEAN PATENT SPECIFICATION

(11) **EP 1 858 851 B1**
(45) Date of publication and mention of the grant of the patent: **24.06.2015**
(21) Application number: 05817765.0
(22) Date of filing: 26.10.2005
(51) Int. Cl.: C07D 211/90

(54) **PREPARING METHOD FOR AMORPHOUS LERCANIDIPINE**
VERFAHREN ZUR HERSTELLUNG VON AMORPHEM LERCANIDIPIN
PROCEDE DE PREPARATION DE LA LERCANIDIPINE AMORPHE

(30) Priority: 27.10.2004 KR 20040086179; 14.04.2005 KR 20050031041
(43) Date of publication of application: 28.11.2007
(73) Proprietor: Recordati Ireland Limited, Ringaskiddy County Cork (IE)
(72) Inventor: CHOO, So Mi, Gyeonggi-do 449-933 (KR); HA, Hong Joo, Gyeonggi-do 449-933 (KR); AN, Jung Gi, Gyeonggi-do 449-933 (KR); LEE, Won Jung, Gyeonggi-do 449-933 (KR); LEE, S. G.,, Gugali Giheung-eup Yongin-si,Gyeonggi-do 449-722 (KR); BYUN, Young-Seok, Kyunggido, 449-931 (KR); MOON, Young Il,, Dongan-ku Anyang-si,Gyeonggi-do 431-070 (KR)
(74) Representative: Serjeants LLP
(86) International application number: PCT/KR2005/003576
(87) International publication number: WO 2006/046830

(56) References cited:
- EP-A- 0 153 016
- US-A- 5 912 351
- US-A1- 2003 083 355
- LEONARDI A ET AL: "Asymmetric N-(3,3-diphenylpropyl)aminoalkyl esters of 4-aryl-2,6-dimethyl-1,4-dihydropyridine-3, 5-dicarboxylic acids with antihypertensive activity" EUROPEAN JOURNAL OF MEDICINAL CHEMISTRY, EDITIONS SCIENTIFIQUE ELSEVIER, PARIS, FR, vol. 33, no. 5, 1 June 1998 (1998-06-01), pages 399-420, XP004127371 ISSN: 0223-5234
- ANSEL H.C.: 'Introduction to pharmaceutical dosage forms', 1985, LEA & FEBIGER, PHILADELPHIA page 69, XP008137504

## Description

### Technical Field

The-present invention relates to a method for preparing amorphous lercanidipine and, more particularly, to a method for preparing amorphous lercanidipine having superior solubility and bioavailability in high purity and yield by controlling major solvents and their volume ratio during the preparation.

### Background Art

Lercanidipine [methyl-1,1,N-trimethyl-N-(3,3-diphenylpropyl)-2-aminoethyl-1,4-dihydro-2,6-dimethyl-4-(3-nitrophenyl)pyridine-3,5-dicarboxylate] and, especially, its hydrochloride salt are one of the latest therapeutic agents for hypertension. They are highly lipophilic d1Hydropyridine calcium antagonists (calcium channel blockers) having high selectivity for blood vessels and exhibiting a long duration of action. They cause vasodilation of the blood vessels and lower blood pressure by acting on calcium channels in cell membranes of the blood vessels. The calcium antagonists suppress contractile force of the heart and lower beat rates thereof by acting on calcium channels in the heart. The calcium antagonists exhibit differences of pharmacokinetic and pharmacological properties because various kinds of calcium antagonists have different physicochemical properties.

In comparison with other calcium antagonists, lercanidipine exhibits a long duration of action in spite of a gradual onset and decrease in the concentration of blood plasma. According to in vitro experiments, even after the removal, of a medicine from the periphery of aorta tissue, the reaction to K⁺ ion of rat's aorta separated for 6 hours is weakened by lercanidipine.

U.S. Patent No. 4,705,797 disclosed that lercanidipine is an L-type calcium channel antagonist and a material used for treatment of angina (such as throat tonsillitis), hypertension and coronary- artery disease.

The preparation methods for lercanidipine are disclosed in U.S. Patent Nos. 5,767,136, 4,968,832, and 5,696,139.

In U.S. Patent No.5,912,351, a more improved preparation method of lercanidipine, is disclosed as follows; 2,6-dimethyl-5-methoxycarbonyl-4-(3-nitrophenyl)-1,4-dihydropyri dine-3-carboxylic acid is halogenated by a halogenating agent in an aprotic solvent, and 2,N-dimethyl-N-(3,3-diphenylpropyl)-1-amino-2-propanol dissolved in aprotic solvent is added to the prepared halide acid to synthesize lercanidipine as an anhydrous hydrochloride.

Lercanidipine is classified into crystal types of I, II, III and IV according to position and intensity of peaks in x-ray diffraction (XRD), and also exists as an amorphous form having no peak in the XRD.

Korea Patent Application No.2004-7001558 discloses a crude form of lercanidipine hydrochloride and a preparation method for novel crystal forms of (I) and (II).

Additionally, Korea Patent Application No.2004-7001791 (European Patent No.1423367) discloses synthesizing methods for novel crystal, forms of (III) and (IV) and pharmaceutical compositions containing at least one of the novel crystal forms of (III) and (IV).

In many pharmaceutical compositions, it is widely known that the amorphous form has different solubility and bioavailability from a crystalline form.

Among therapeutic indicators, bioavailability is one of the most important parameters for determining a form of a substance used for pharmacological formulation.

Generally, the solubility of a crystalline form in an organism is lower than that of an amorphous form. Hydrochloride lercanidipine has extremely low water solubility, and its crystalline form has lower solubility than the amorphous form thereof, and thereby it may cause problems in a bioavailability of the lercanidipine. Additionally, an amorphous form causes big problems infiltration because the amorphous form has a small particle size and large surface area.

Thus, the inventors have completed a method for preparing novel amorphous lercanidipine compound that does not belong to crystalline forms of (I), (II), (III) or (IV). The solubility, bioavailability and filtering property may be improved by transforming a crystalline form of the lercanidipine into an amorphous form.

### Disclosure of the Invention

### Technical Problem

An object of the present invention is to provide a method for preparing amorphous lercanidipine having an excellent solubility, bioavailability and filtering property.

### Technical Solution

In order to achieve the above object, the present invention provides a method for preparing amorphous lercanidipine comprising the steps of: (1) dissolving lercanidipine in an organic solvent to prepare a solution; (2) forming precipitates by dropping the solution into an organic solvent selected from the group consisting of cyclohexane, cyclopentane, hexane, and petroleum ether and stirring; and (3) filtering the precipitates, washing the precipitates with the organic solvent used in step (2) and vacuum drying.

The present invention provides another method for preparing amorphous lercanidipine comprising the steps of: (1) dissolving lercanidipine in an organic solvent selected from the group consisting of dimethylsulfoxide, dimethylformamide, 1,4-dioxane, and methanol and cooling to 0 °C; (2) forming precipitates by adding water of 0 ∼ 5 °C and stirring; and (3) filtering the precipitates, washing with water and vacuum drying.

The present invention provides another method for preparing amorphous lercanidipine comprising the steps of: (1) dissolving lercanidipine in methanol and adding diisopropyl ether to obtain a dilute solution; (2) forming precipitates by adding the solution into diisopropyl ether and stirring; and (3) filtering the precipitates, washing the precipitates with diisopropyl ether and vacuum drying.

Hereinafter, the present invention will be described in detail. A method for preparing amorphous lercanidipine according to the present invention comprises the steps of: (1) dissolving lercanidipine in an organic solvent to prepare a solution; (2) forming precipitates by dropping the solution into an organic solvent selected from the group consisting of cyclohexane, cyclopentane, hexane, and petroleum ether and stirring; and (3) filtering the precipitates, washing the precipitates with the organic solvent used in step (2) and vacuum drying.

In step (1) of the present invention, the organic solvent is used to uniformly dissolve lercanidipine. As examples, a mixed organic solvent of methylene chloride and t-butyl alcohol or a mixed organic solvent of tetrahydrofuran and t-butyl alcohol may be used as the organic solvent. When the mixed organic solvent of methylene chloride and t-butyl alcohol is used, lercanidipine is dissolved in the mixed organic solvent 4 to 10 times of the lercanidipine's volume, preferably in the mixed organic solvent 6 times of the lercanidipine's volume, and then in step (2), the solution is dropped into cyclohexane 15 to 40 times of the methylene chloride's volume, preferably into cyclohexane 20 times of the methylene chloride's volume.
Step (1) of the present invention may comprise the additional steps of: uniformly dissolving lercanidipine in at least one organic solvent selected from the group consisting of methanol, ethanol, and methylene chloride to prepare a solution; evaporating the solution to obtain a solid substance; and dissolving the solid substance in a solvent of tetrahydrofuran. The volume of tetrahydrofuran is preferably 5 to 10 times the volume of lercanidipine used, more preferably 8 times. Amorphous form of the lercanidipine is obtained by the addition of tetrahydrofuran more than 5 times of the lercanidipine's volume. However the use of tetrahydrofuran more than 10 times of the lercanidipine's volume is not economical because yield is not significantly increased in spite of increased use of the tetrahydrofuran.
In step (2) of the present invention, the volume of an organic solvent selected from the group consisting of cyclohexane, cyclopentane, hexane, and petrole.um ether is 5 to 40 times the volume of the organic solvent used to dissolve the lercanidipine, preferably 7 to 10 times. Amorphous form of the lercanidipine is obtained by adding a volume of the organic solvent more than 5 times the volume of the organic solvent used to dissolve the lercanidipine. When the volume of-the organic solvent used is more than 40 times the volume of the organic solvent used to dissolve the lercanidipine, the amorphous form of the lercanidipine is obtained but it is not economical because yield is not significantly increased in spite of excess use of the organic solvent. However, if the volume of the organic solvent used is less than 5 times the volume of the organic solvent used to dissolve the lercanidipine, formation of crystalline form may occur.

In step (2) of the present invention, the dropping operation may be carried out at 0°C. At this temperature, crystal formation of lercanidipine is sufficiently suppressed and this temperature is preferable to obtain an amorphous lercanidipine. Cyclopentane or petroleum ether may be used in this step.

In step (3) of the present invention, filtration should preferably be carried out as soon as possible after the formation of precipitates in step (2). As the time after the formation of the precipitates increases, the possibility of the formation of high melting-point amorphous form or crystalline form become higher. Therefore, the filtration should preferably be carried out within about 2 hours after the step of solution mixing. Further, as the time decreases, the yield of low melting-point amorphous lercanidipine becomes higher.

In step (3) of the present invention, the vacuum drying may be carried out at 60 ~ 80 °C, preferably at 70 °C, for 24 ~ 30 hours. When the drying temperature is less than 60 °C, the drying rate is so slow that high melting-point amorphous form and crystalline form may be formed together. When the drying temperature is more than 80 °C, poor quality product such as colored product may be formed due to melting or denaturation, and therefore a high drying temperature is not preferable.

Another method for preparing amorphous lercanidipine comprises the steps of: (1) dissolving lercanidipine in at least one organic solvent selected from the group consisting of dimethylsulfoxide, dimethylformamide, 1,4-dioxane and methanol, and cooling to 0 °C; (2) forming precipitates by adding water of 0 ~ 5 °C and stirring; and (3) filtering the precipitates, washing with water and vacuum drying.

In step (1) of the present invention, the volume of the organic solvent used is preferably 2 to 5 times the volume of the lercanidipine, preferably 2 to 3 times. When the volume of the organic solvent used is less than 2 times the volume of lercanidipine, coagulation occurs. When the volume of the organic solvent used exceeds 5 times the volume of lercanidipine, the cooling time is increased. The melting point of the amorphous lercanidipine is high and there by its in vivo solubility and bioavailability are decreased.

In step (2) of the present invention, the preparation of water of 0 ~ 5 °C has an advantage in preparing that the chilled water can be easily prepared by adding ice into water without special temperature control. The volume of the prepared water is preferably 15 to 30 times the volume of the solvent used to dissolve the lercanidipine, more preferably 20 times. Crystalline forms are mixed during the formation of precipitates when the volume of added water is less than 15 times the volume of the solvent used to dissolve the lercanidipine. When the volume of added water exceeds 30 times the volume of the solvent used to dissolve the lercanidipine, it is not economical because yield is not significantly increased in spite of additional water consumption.

In step (3) of the present invention, vacuum drying may be carried out at 45 ~ 55 °C, preferably at 50 °C, for 18 ~ 22 hours, more preferably for 20 hours.

Another method for preparing amorphous lercanidipine comprises the steps of: (1) dissolving lercanidipine in methanol and adding diisopropyl ether thereto to obtain a dilute solution; (2) forming precipitates by adding the solution into diisopropyl ether, and stirring; and (3) filtering the precipitates, washing with diisopropyl ether and vacuum drying.

In the present invention, if there is no additional in step (1) after lercanidipine is dissolved in methanol, excessive coagulation occurs. The coagulation may be prevented by addition a small amount of diisopropyl ether for weak dilution. The volume of diisopropyl ether to obtain a dilute solution in step 1 is preferably twice the volume of methanol used for dissolving lercanidipine.

In step (3) of the present invention, the volume of diisopropyl ether is preferably 30 to 50 times the volume of lercanidipine, preferably 40 times. Amorphous form of the lercanidipine is obtained when the volume of diisopropyl ether is more than 30 times the volume of the lercanidipine. When the volume of diisopropyl ether is more than 50 times the volume of the lercanidipine, the amorphous form of the lercanidipine is obtained but it is not economical because yield is not significantly increased in spite of increased use of diisopropyl. If the volume of the organic solvent used is less than 30 times the volume of the lercanidipine, formation of crystalline form may occur.

In step (3) of the present invention, the vacuum drying is carried out at 60 ~ 80 °C, preferably at 70 °C, for 24 ~ 30 hours.

### Advantageous Effect

A method for preparing amorphous lercanidipine according to the present invention has an advantage that additional investment in equipments is not required because the method does not require equipments for rapidly evaporating solvents and for maintaining reduced pressure to remove solid media by sublimation. Amorphous lercanidipine having excellent filtering property, superior solubility and bioavailability may be prepared in high purity and yield by properly controlling major solvents and reaction temperature thereof.

### Brief Description of Drawings

FIG. 1 shows an XRD (X-ray Diffraction) graph of amorphous lercanidipine obtained by using cyclohexane as a solvent according to an example embodiment of the present invention.
FIG. 2 shows an XRD (X-ray Diffraction) graph of amorphous lercanidipine obtained by using cyclopentane as a solvent according to an example embodiment of the present invention.
FIG. 3 shows an XRD (X-ray Diffraction) graph of amorphous lercanidipine obtained by using petroleum ether as a solvent according to an example embodiment of the present invention.
FIG. 4 shows an XRD (X-ray Diffraction) graph of amorphous lercanidipine obtained by using dimethylsulfoxide as a solvent according to an example embodiment of the present invention.
FIG. 5 shows an XRD (X-ray Diffraction) graph of amorphous lercanidipine obtained by using dimethylformamide as a solvent according to an example embodiment of the present invention.
FIG. 6 shows an XRD (X-ray Diffraction) graph of amorphous lercanidipine obtained by using 1,4-dioxane as a solvent according to an example embodiment of the present invention.
FIG. 7 shows an XRD (X-ray Diffraction) graph of amorphous lercanidipine obtained by using methanol as a solvent according to an example embodiment of the present invention.
FIG. 8 shows an XRD (X-ray Diffraction) graph of amorphous lercanidipine obtained using diisopropyl ether as a solvent according to an example embodiment of the present invention.

### Best Mode for Carrying Out the Invention

Hereinafter, preferred example embodiments of the present invention will be described more fully. This invention may, however, be embodied in many different forms and should not be construed as limited to the example embodiments set forth herein.

### <Example 1> Preparation of amorphous lercanidipine

1 g of lercanidipine (mixture of R and S isomers) is dissolved in methanol and a solid substance is obtained after complete evaporation. After the solid substance is dissolved in 8 mL of tetrahydrofuran to prepare a solution, the solution is slowly added dropwise into 56 mL of cyclohexane and stirred for 5 mins. Formed precipitates are filtered and washed with 10 mL of cyclohexane 99 mg of amorphous lercanidipine is obtained by drying the precipitates at 70 °C for 30 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 7.45-7.3 ((m, 1H), δ 7.30-7.19 (m, 10H), δ 8.0-6.0 (two s, 1H), δ 5.00(d, 1H), δ 3.90-3.84 (m, 1H), δ 3.77-3.24(m, 2H), δ 3.67(d, 3H), δ 3.01-2.91 (m, 2H), δ 3.03-2.89(m, 2H), δ 2.40-2.26(m, 6H), δ 1.65-1.49 (m, 6H).

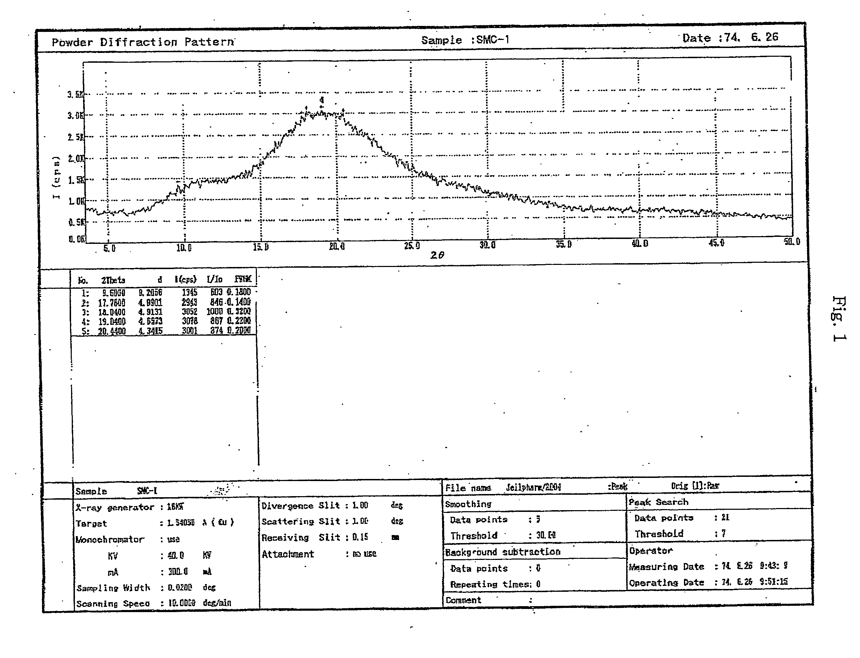

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 1).

### <Example 2> Preparation of amorphous lercanidipine

1 g of lercanidipine (mixture of R and S isomers) is dissolved in methanol and a solid substance is obtained after complete evaporation. After the solid substance is dissolved in 8 mL of tetrahydrofuran to prepare a solution, the solution is slowly added dropwise into 56mL of cyclopentane at 0 °C and stirred for 5 mins. Formed precipitates are filtered and washed with 10 mL of cyclopentane. 99 mg of amorphous lercanidipine is obtained by drying the precipitates at 70 °C for 30 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 1.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), 6 2.40-2.26 (m, 6H), δ 1.65-1.49 (m, 6H).

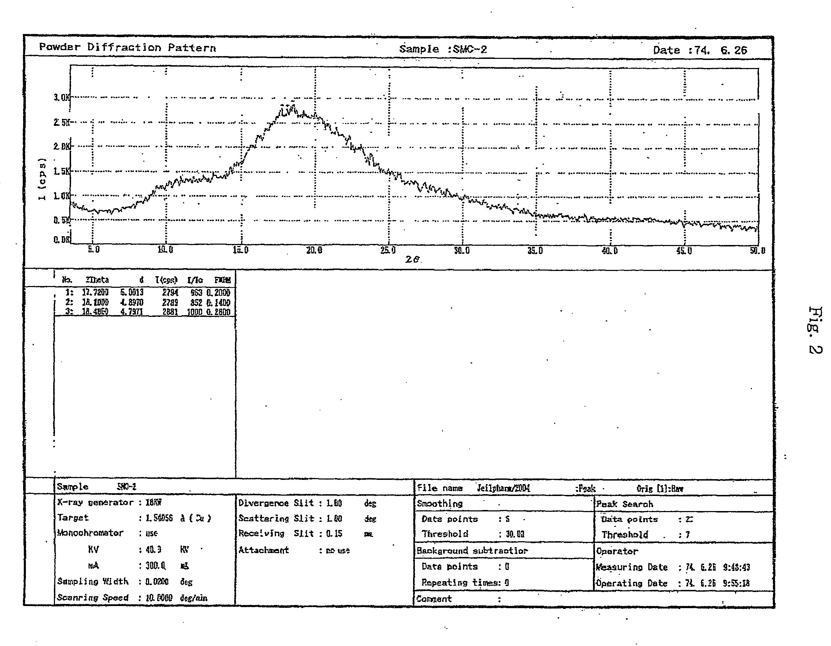

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 2).

### <Example 3> Preparation of amorphous lercanidipine

1 g of lercanidipine (mixture of R and S isomers) is dissolved in methanol and a solid substance is obtained after complete evaporation. After the solid substance is dissolved in 8 mL of tetrahydrofuran to prepare a solution, the solution is slowly added dropwise into 56mL of hexane and stirred for 5mins. Formed precipitates are filtered and washed with 10 mL of hexane. 99 mg of amorphous lercanidipine is obtained by drying the precipitates at 70 °C for 30 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 1.45-7.34 ((m, 1H), δ 7.30-7.19 (m, 10H), δ 8.0-6.0 (two-s, 1H), δ 5.00 (d, 1H), δ 3.90-3.84 (m, 1H), δ 3.77-3.24 (m, 2H), δ 3.67(d, 3H), δ 3.01-2.91 (mf 2H), δ 3.03-2.89 (m, 2H), δ 2.40-2.26 (m, 6H), δ 1.65-1.49 (m, 6H).

### <Example 4> Preparation of amorphous lercanidipine

1 g of lercanidipine (mixture of R and S isomers) is dissolved in methanol and a solid substance is obtained after complete evaporation. After the solid substance is dissolved in 8 mL of tetrahydrofuran to prepare a solution, the solution is slowly added dropwise into 56 mL of petroleum ether at 0 °C and stirred for 5 mins. Formed precipitates are filtered and washed with 10 mL of petroleum ether. 99mg of amorphous lercanidipine is obtained by drying the precipitates at 70 °C for 30 hrs under vacuum condition.

HPLC analysis:
Amorphous lercanidipine: 99.9 %

H¹-NMR (CDCl₃, ppm): δ 11.7 (bd, 1H), 6 8.08-8.05 (m, 1H), δ 8.08-7.97 (m, 1H), 7.62-7.59 (m, 1H), δ 7.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

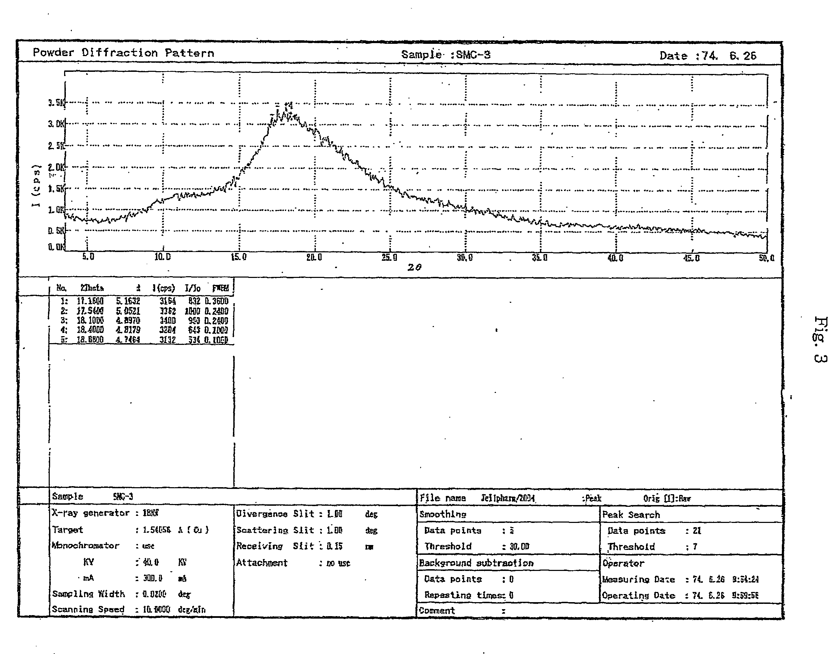

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 3).

### <Example 5> Preparation of amorphous lercanidipine

After 1 g of lercanidipine (mixture of R and S isomers) is dissolved in a mixed solution of tetrahydrofuran (7 mL) and t-butyl alcohol (1 mL) to prepare a solution, the solution is slowly added dropwise into 56 mL of cyclohexane and stirred for 5 mins. Formed precipitates are filtered and washed with 10 mL of cyclohexane. 99 mg of amorphous lercanidipine is obtained by drying the precipitates at 70 °C for 24 hrs under vacuum condition.

HPLC analysis:
Amorphous lercanidipine: 99.9 %

H¹-NMR (CDCl₃, ppm): δ 11.7 (bd, 1H),- δ 8.08-8.05 (m, 1H), δ 8.08-7.97 (m, 1 H), 7.62-7.59 (m, 1H), δ 1.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

### <Example 6> Preparation of amorphous lercanidipine

After 1 g of lercanidipine (mixture of R and S isomers) is dissolved in a mixed solution of methylenechloride (5.25 mL) and t-butyl alcohol (0.75 mL) to prepare a solution, the solution is slowly added dropwise into 120 mL of cyclohexane and stirred for 5 mins. Formed precipitates are filtered and washed with 10 mL of cyclohexane. 99 mg of amorphous lercanidipine is obtained by drying the precipitates at 70 °C for 24 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 1.45-7.34 ((m, 1H), δ 7.30-7.19 (m, 10H), δ 8.0-6.0 (two s, 1H), δ 5.00(d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

### <Example 7> Preparation of amorphous lercanidipine

1 g of lercanidipine (mixture of R and S isomers) is dissolved in 3 mL of dimethylsulfoxide to prepare a solution and cooled at 0 °C. The solution is added with 60 mL of water at a temperature of 0 ~ 5 °C and stirred for 15 mins. Formed precipitates are filtered and washed with 30 mL of water. 98mg of amorphous lercanidipine is obtained by drying the precipitates at 50 °C for 20 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 7.45-7.34 ((m, 1H), δ 7.30-7.19 (m, 10H), δ 8.0-6.0 (two s, 1H), δ 5.00 (d, 1H), δ 3.90-3.84(m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

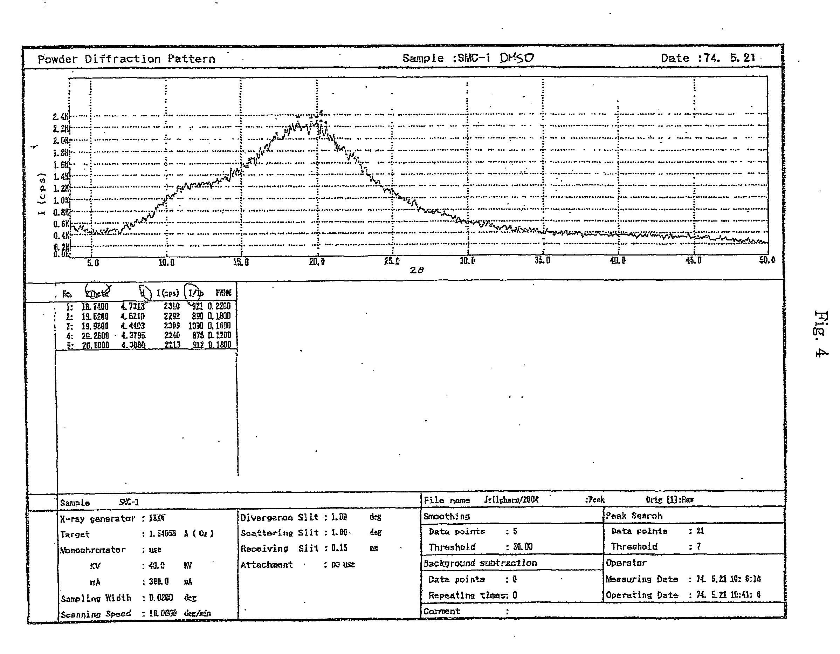

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 4).

### <Example 8> Preparation of amorphous lercanidipine

1 g of lercanidipine (mixture of R and S isomers) is dissolved in 3 mL of dimethylformamide to prepare a solution and cooled to 0 °C. The solution is added with 60 mL of water at a temperature of 0 ~ 5 °C and stirred for 15 mins. Formed precipitates are filtered and washed with 30 mL of water. 98 mg of amorphous lercanidipine is obtained by drying the precipitates at 50 °C for 20 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 1.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

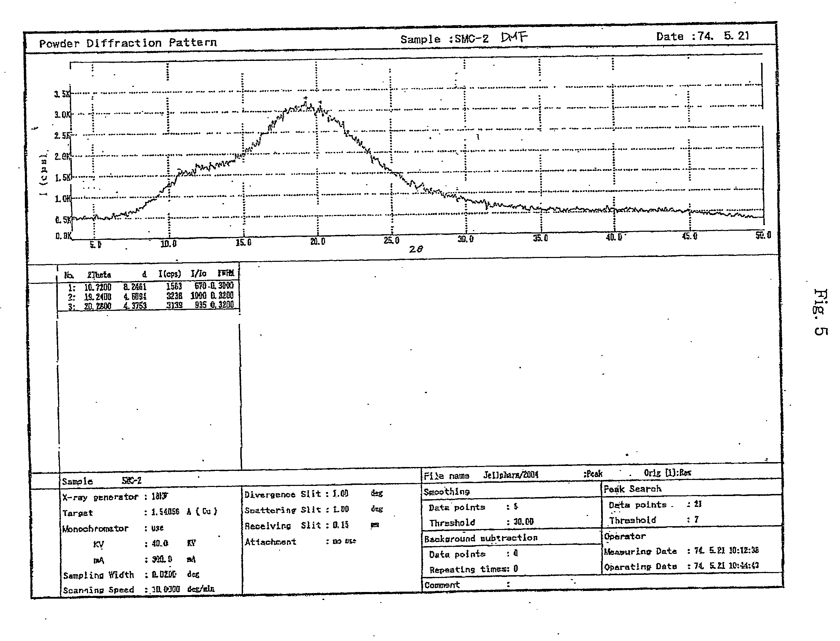

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 5).

### <Example 9> Preparation of amorphous lercanidipine

1 g of lercanidipine (mixture of R and S isomers) is dissolved in 3 mL of 1,4-dioxane to prepare a solution and cooled to 0 °C . The solution is added with 60 mL of 0 ~ 5 °C water and stirred for 15 mins. Formed precipitates are filtered and washed with 30 mL of water. 98 mg of amorphous lercanidipine is obtained by drying the precipitates at 50 °C for 20 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 7.45-7.34 ((m, 1H), δ 7.30-7.19 (m, 10H), δ 8.0-6.0 (two s, 1H), δ 5.00(d, 1H), δ 3.90-3.84(m, 1H); δ 3.77-3.24 (m, 2H), δ 3.67(d, 3H), δ 3.01-2.91 (m, 2H), δ 3.03-2.89 (m, 2H), δ 2.40-2.26 (m, 6H), δ 1.65-1.49 (m, 6H).

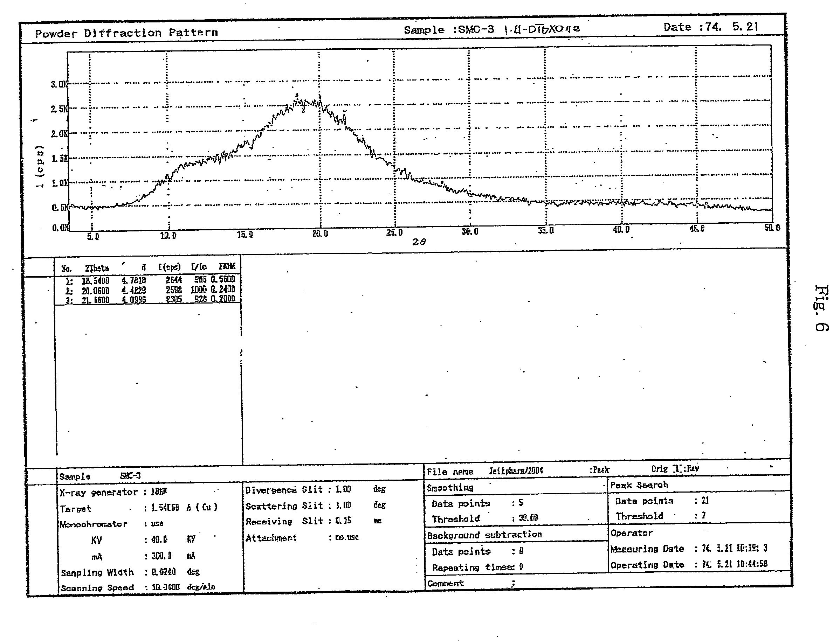

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 6).

### <Example 10> Preparation of amorphous lercanidipine

1 g of lercanidipine (mixture of R and S isomers) is dissolved in 3 mL of methanol to prepare a solution and cooled to 0 °C. The solution is added with 60 mL of 0 ~ 5 °C water and stirred for 15 mins. Formed precipitates are filtered and washed with 30 mL of water. 98 mg of amorphous lercanidipine is obtained by drying the precipitates at 50 °C for 20 hrs under vacuum condition.

HPLC analysis:
Amorphous lercanidipine: 99.9 %

H¹-NMR (CDCl₃, ppm): δ 11.7 (bd, 1H), δ 8.08-8.05 (m, 1H), δ 8.08-7.97 (m, 1H), 7.62-7.59 (m, 1H), δ 1.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67 (d, 3H), δ 3.01-2.91 (m, 2H), δ 3.03-2.89 (m, 2H), δ 2.40-2.26 (m, 6H), δ 1.65-1.49 (m, 6H).

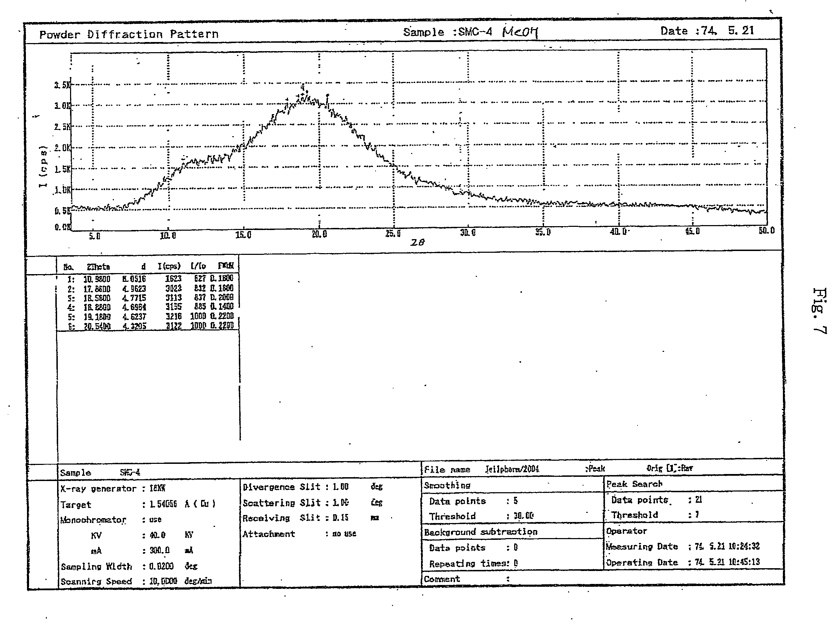

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 7).

### <Example 11> Preparation of amorphous lercanidipine

1 g of lercanidipine (mixture of R and S isomers) is dissolved in 5 mL of methanol, and 10 mL of diisopropyl ether is added to prepare a dilute solution. The solution is added into 140 mL of diisopropyl ether and stirred for 5 mins. Formed precipitates are filtered and washed with 10 mL of diisopropyl ether. 99 mg of amorphous lercanidipine is obtained by drying the precipitates at 70 °C for 30 hrs under vacuum condition.

HPLC analysis:
Amorphous lercanidipine: 99.9 %

H¹-NMR (CDCl₃, ppm): δ 11.7 (bd, 1H), δ 8.08-8.05 (m,. 1H), δ 8.08-7.97 (m, 1H), 7.62-7.59 (m, 1H), δ 7.45-7.34 ((m, 1H), δ 7.30-7.19 (m, 10H), δ 8.0-6.0 (two s, 1H), δ 5.00 (d, 1H), δ 3.90-3.84 (m, 1H), δ 3.77-3.24 (m, 2H), δ 3.67(d, 3H), δ 3.01-2.91 (m, 2H), δ 3.03-2.89 (m, 2H), δ 2.40-2.26 (m, 6H), δ 1.65-1.49 (m, 6H).

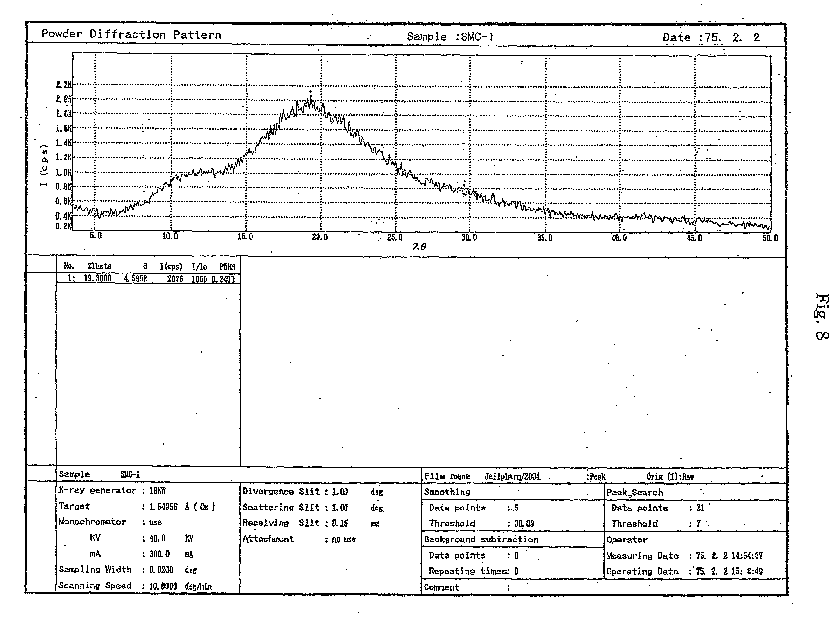

XRD (Mac Science M18XHF(18KW) power diffractometer): Power for sample measurement is 40 KV and 300 mA, and diffraction peaks are obtained at a scan rate of 10°/min. The shapes of the obtained peaks show halo of a typical amorphous form that is completely different from that of a crystalline form. From the XRD measurement, it is identified that the product is amorphous lercanidipine having no crystalline form (refer to FIG. 8).

As described above, in the preparation of amorphous lercanidipine, it is identified that the kinds and volume ratio of solvents, temperature and, time are very important factors for determining the form of the product.

According to the present invention, a target compound of amorphous lercanidipine may be obtained in a high yield of above 99 % and with high reproducibility.

It is difficult to filter amorphous form because it has a small- particle size and large surface area. However, the lercanidipine prepared according to the present invention provides an advantage in filtration by increasing a particle size of amorphous form. It is identified that the lercanidipine prepared according to the present invention is atypical amorphous form showing no peak in XRD diffraction data.

### Industrial Applicability

A method for preparing amorphous lercanidipine according to the present invention has an advantage that any additional investment in equipments is not required because the method does not need equipments for rapidly evaporating solvents and for maintaining reduced pressure to remove solid media by sublimation compared with a conventional method for preparing lercanidipine. Amorphous lercanidipine having an excellent filtering property, solubility and bioavailability may be prepared in high purity and yield by properly controlling major solvents and reaction temperature, and may be utilized usefully in the industry.

## Claims

1. A method for preparing amorphous lercanidipine comprising the steps of:
(1) dissolving lercanidipine in an organic solvent to prepare a solution;
(2) forming precipitates by dropping the solution into an organic solvent selected from the group consisting of cyclohexane, cyclopentane, hexane and petroleum ether and stirring; and
(3) filtering the precipitates, washing with the organic solvent used in step 2 and vacuum drying.

2. The method of claim 1, wherein the organic solvent of step (1) is a mixed solution of methylene chloride and t-butyl alcohol or a mixed solution of tetrahydrofuran and t-butyl alcohol.

3. The method of claim 1, wherein step (1) further comprises the steps of: dissolving the lercanidipine in at least one organic solvent selected from the group consisting of methanol, ethanol and methylene chloride to prepare a solution; evaporating the solution to obtain a solid substance; and dissolving the solid substance in tetrahydrofuran.

4. The method of claim 3, wherein the volume of the tetrahydrofuran is 5 to 10 times the volume of the lercanidipine.

5. The method of claim 1, wherein the volume of the organic solvent used in step (2) is 5 to 40 times the volume of the solvent used to dissolve the lercanidipine.

6. The method of claim 1, wherein the organic solvent of step (1) is a mixed organic solvent of methylene chloride and t-butyl alcohol, the volume of the mixed organic solvent being 4 to 10 times the volume of the lercanidipine; the organic solvent of step (2) is cyclohexane having a volume 15 to 40 times the volume of the organic solvent of step (1); and the vacuum drying in step (3) takes place at 60 ~ 80 °C for 24 ~ 30 hrs.

7. The method of claims 1 or 6, wherein the dropping in step (2) is carried out at 0 °C.

8. A method for preparing amorphous lercanidipine comprising the steps of:
(1) dissolving lercanidipine in at least one organic solvent selected from the group consisting of dimethylsulfoxide, dimethylformamide, 1,4-dioxane and methanol, and cooling to 0 °C;
(2) forming precipitates by adding water of 0 ~ 5 °C and stirring; and
(3) filtering the precipitates, washing with water, and vacuum drying.

9. The method of claim 8, wherein the volume of the organic solvent in step (1) is 2 to 5 times the volume of the lercanidipine.

10. The method of claim 8, wherein the volume of the water in step (2) is 15 to 30 times the volume of the organic solvent used to dissolve the lercanidipine.

11. A method for preparing amorphous lercanidipine comprising the steps of:
(1) dissolving lercanidipine in methanol and adding diisopropyl ether to obtain a dilute solution;
(2) forming precipitates by adding the solution into diisopropyl ether and stirring; and
(3) filtering the precipitates, washing with diisopropyl ether and vacuum drying.

12. The method of claim 11; wherein the volume of the diisopropyl ether step (3) is 30 to 50 times the volume of the lercanidipine.

## Patentansprüche

1. Ein Verfahren zur Herstellung von amorphem Lercanidipin, umfassend die Schritte:
(1) Lösen von Lercanidipin in einem organischen Lösungsmittel, um eine Lösung herzustellen;
(2) Bilden von Ausfällungen durch Tropfen der Lösung in ein organisches Lösungsmittel, ausgewählt aus der Gruppe bestehend aus Cyclohexan, Cyclopentan, Hexan und Petrolether, und Rühren; und
(3) Filtrieren der Ausfällungen, Waschen mit dem in Schritt 2 verwendeten organischen Lösungsmittel und Vakuumtrocknen.

2. Das Verfahren nach Anspruch 1, wobei das organische Lösungsmittel von Schritt (1) eine gemischte Lösung aus Methylenchlorid und t-Butylalkohol oder eine gemischte Lösung aus Tetrahydrofuran und t-Butylalkohol ist.

3. Das Verfahren nach Anspruch 1, wobei Schritt (1) ferner die Schritte umfasst: Lösen des Lercanidipins in mindestens einem organischen Lösungsmittel, ausgewählt aus der Gruppe bestehend aus Methanol, Ethanol und Methylenchlorid, um eine Lösung herzustellen, Verdampfen der Lösung, um eine feste Substanz zu erhalten, und Lösen der festen Substanz in Tetrahydrofuran.

4. Das Verfahren nach Anspruch 3, wobei das Volumen des Tetrahydrofurans das 5- bis 10-Fache des Volumens des Lercanidipins beträgt.

5. Das Verfahren nach Anspruch 1, wobei das Volumen des in Schritt (2) verwendeten organischen Lösungsmittels das 5- bis 40-Fache des Volumens des zur Lösung des Lercanidipins verwendeten Lösungsmittels beträgt.

6. Das Verfahren nach Anspruch 1, wobei das organische Lösungsmittel von Schritt (1) ein gemischtes organisches Lösungsmittel aus Methylenchlorid und t-Butylalkohol ist, wobei das Volumen des gemischten organischen Lösungsmittels das 4- bis 10-Fache des Volumens des Lercanidipins beträgt, das organische Lösungsmittel von Schritt (2) Cyclohexan ist mit einem Volumen, welches das 15- bis 40-Fache des Volumens des organischen Lösungsmittels von Schritt (1) beträgt, und das Vakuumtrocknen in Schritt (3) bei 60 ~ 80 °C für 24 ~ 30 Stunden stattfindet.

7. Das Verfahren nach den Ansprüchen 1 oder 6, wobei das Tropfen in Schritt (2) bei 0 °C durchgeführt wird.

8. Ein Verfahren zur Herstellung von amorphem Lercanidipin, umfassend die Schritte:
(1) Lösen von Lercanidipin in mindestens einem organischen Lösungsmittel, ausgewählt aus der Gruppe bestehend aus Dimethylsulfoxid, Dimethylformamid, 1,4-Dioxan und Methanol, und Abkühlen auf 0 °C;
(2) Bilden von Ausfällungen durch Zugeben von Wasser mit 0 ~ 5 °C und Rühren; und
(3) Filtrieren der Ausfällungen, Waschen mit Wasser und Vakuumtrocknen.

9. Das Verfahren von Anspruch 8, wobei das Volumen des organischen Lösungsmittels in Schritt (1) das 2- bis 5-Fache des Volumens des Lercanidipins beträgt.

10. Das Verfahren nach Anspruch 8, wobei das Volumen des Wassers in Schritt (2) das 15- bis 30-Fache des Volumens des zum Lösen des Lercanidipins verwendeten organischen Lösungsmittels beträgt.

11. Ein Verfahren zur Herstellung von amorphem Lercanidipin, umfassend die Schritte:
(1) Lösen von Lercanidipin in Methanol und Zugeben von Diisopropylether, um eine verdünnte Lösung zu erhalten;
(2) Bilden von Ausfällungen durch Zugeben der Lösung zu Diisopropylether und Rühren; und
(3) Filtrieren der Ausfällungen, Waschen mit Diisopropylether und Vakuumtrocknen.

12. Das Verfahren nach Anspruch 11, wobei das Volumen des Diisopropylether Schritt (3) das 30- bis 50-Fache des Volumens des Lercanidipins beträgt.

## Revendications

1. Procédé de préparation de lercanidipine amorphe comprenant les étapes de:
(1) dissoudre de la lercanidipine dans un solvant organique pour préparer une solution ;
(2) former des précipités en versant la solution dans un solvant organique choisi dans le groupe constitué du cyclohexane, du cyclopentane, de l'hexane et de l'éther de pétrole, et agiter ; et
(3) filtrer les précipités, les laver avec le solvant organique utilisé dans l'étape 2 et sécher sous vide.

2. Procédé selon la revendication 1, dans lequel le solvant organique de l'étape (1) est une solution mixte de chlorure de méthylène et d'alcool t-butylique ou une solution mixte de tétrahydrofuranne et d'alcool t-butylique.

3. Procédé selon la revendication 1, dans lequel l'étape (1) comprend en outre les étapes consistant à : dissoudre la lercanidipine dans au moins un solvant organique choisi dans le groupe constitué du méthanol, de l'éthanol et du chlorure de méthylène pour préparer une solution ; évaporer la solution pour obtenir une substance solide ; et dissoudre la substance solide dans du tétrahydrofuranne.

4. Procédé selon la revendication 3, dans lequel le volume du tétrahydrofuranne est 5 à 10 fois le volume de la lercanidipine.

5. Procédé selon la revendication 1, dans lequel le volume du solvant organique utilisé dans l'étape (2) est 5 à 40 fois le volume du solvant utilisé pour dissoudre la lercanidipine.

6. Procédé selon la revendication 1, dans lequel le solvant organique de l'étape (1) est un solvant organique mixte de chlorure de méthylène et d'alcool t-butylique, le volume du solvant organique mixte étant 4 à 10 fois le volume de la lercanidipine ; le solvant organique de l'étape (2) est du cyclohexane ayant un volume 15 à 40 fois le volume du solvant organique de l'étape (1) ; et le séchage sous vide de l'étape (3) a lieu à 60 à 80 °C pendant 24 à 30 heures.

7. Procédé selon les revendications 1 ou 6, dans lequel le versage de l'étape (2) est réalisé à 0 °C.

8. Procédé de préparation de lercanidipine amorphe comprenant les étapes de:
(1) dissoudre de la lercanidipine dans au moins un solvant organique choisi dans le groupe constitué du diméthylsulfoxyde, du diméthylformamide, du 1,4-dioxane et du méthanol, et refroidir à 0 °C ;
(2) former des précipités par addition d'eau à 0 à 5 °C et agiter ; et
(3) filtrer les précipités, les laver avec de l'eau, et sécher sous vide.

9. Procédé selon la revendication 8, dans lequel le volume du solvant organique de l'étape (1) est 2 à 5 fois le volume de la lercanidipine.

10. Procédé selon la revendication 8, dans lequel le volume de l'eau de l'étape (2) est 15 à 30 fois le volume du solvant organique utilisé pour dissoudre la lercanidipine.

11. Procédé de préparation de lercanidipine amorphe comprenant les étapes de :
(1) dissoudre de la lercanidipine dans du méthanol et ajouter de l'éther diisopropylique pour obtenir une solution diluée ;
(2) former des précipités en ajoutant la solution dans de l'éther diisopropylique et agiter ; et
(3) filtrer les précipités, les laver avec de l'éther diisopropylique et sécher sous vide.

12. Procédé selon la revendication 11 ; dans lequel le volume de l'éther diisopropylique dans l'étape (3) est 30 à 50 fois le volume de la lercanidipine.
